# FASCICULE DE BREVET EUROPEEN

(11) **EP 2 087 093 B1**
(45) Date de publication et mention de la délivrance du brevet: **17.04.2013**
(21) Numéro de dépôt: 07858425.7
(22) Date de dépôt: 11.10.2007
(51) Int. Cl.: C12N 1/12, A61K 36/02, A61P 35/00

(54) **INTEGRATION DE MOLECULES ACTIVES DANS DES MICRO ALGUES**
EINFÜGUNG VON AKTIVMOLEKÜLEN IN MIKROALGEN
INCORPORATION OF ACTIVE MOLECULES INTO MICROALGAE

(30) Priorité: 19.10.2006 FR 0609145
(43) Date de publication de la demande: 12.08.2009
(73) Titulaire: Oreal, Henri, 83500 La Seyne sur Mer (FR); Brune, Jean-Pierre, 83160 La Valette (FR)
(72) Inventeur: Oreal, Henri, 83500 La Seyne sur Mer (FR); Brune, Jean-Pierre, 83160 La Valette (FR)
(74) Mandataire: Cabinet Plasseraud
(86) Numéro de dépôt international: PCT/FR2007/001660
(87) Numéro de publication internationale: WO 2008/046985

(56) Documents cités:
- KHAN MAHMOOD ET AL: "Protective effect of Spirulina against doxorubicin-induced cardiotoxicity" PHYTOTHERAPY RESEARCH, vol. 19, no. 12, décembre 2005 (2005-12), pages 1030-1037, XP002448604 ISSN: 0951-418X
- KHAN MAHMOOD ET AL: "Spirulina attenuates cyclosporine-induced nephrotoxicity in rats" JOURNAL OF APPLIED TOXICOLOGY, vol. 26, no. 5, septembre 2006 (2006-09), pages 444-451, XP002448605 ISSN: 0260-437X
- RYAN RACHEL M ET AL: "Use of bacteria in anti-cancer therapies" BIOESSAYS, vol. 28, no. 1, janvier 2006 (2006-01), pages 84-94, XP002448606 ISSN: 0265-9247

## Description

La présente invention décrit un procédé permettant d'ajouter des molécules actives dans un milieu de cultures pour micro-algues, afin de proposer des vecteurs de molécules permettant une diminution considérable des effets secondaires de la chimiothérapie tout en maintenant celle-ci à son plus haut niveau de biodisponibilité.

En temps normal, les molécules cyto-toxiques comme les anti-métabolites qui agissent en empêchant la synthèse de différentes molécules impliquées dans le système DNA et les agents alkylants qui sont des molécules contenant des groupes akyls ainsi que les extraits de micro-organismes comme la daunorubicine entraînent l'apopthose des cellules cancéreuses ; malheureusement tous ces agents ne sont pas spécifiques de la cellule cancéreuse et des cellules dites normales sont considérablement touchées et en particulier la lignée des cellules sanguines.

Ces vecteurs choisis sont des cyanobactéries.

Les médicaments et les molécules ne sont pas associés mais ajoutés dans les micro-algues.

Les molécules seront essentiellement des anti-cancéreux et parmi les anti-cancéreux choisis nous pouvons citer le taxoter, les inhibiteurs de kinases, le flavopiridol, la roscovitine, l'indoxan, la doxorubicine ou la ricine A etc....

Ces molécules actives par leur composition induisent un dysfonctionnement au niveau de la diffusion cellulaire du vecteur, ils ne peuvent donc être utilisés en tant que tel.

Parmi les micro-algues choisis nous utiliseront essentiellement la spiruline (arthrospira platensis) qui a fait l'objet d'une autorisation de consommation en matière humaine et animale de la part du conseil de l'hygiène publique en France.

Cette cyanobactérie a elle seule a des actions connues sur l'organisme humain. Elle est très protéinée (65%), contient des vitamines, des minéraux, des polysaccharides, des peptides, des pigments.

Par ailleurs elles ont des fonctions importantes notamment de pouvoir fixer naturellement des molécules par des procédés ionisables ou chimiques, mais en ce qui concerne les molécules anti-cancéreuses elles ne peuvent induire ou fixer de telles molécules agressives, sans en avoir exprimé une éventuelle sélection de forme mutante de cyanobactérie et qui représente une résistance à ces molécules anticancéreuse.

L'invention a pour objet de développer une sélection éventuelle de forme mutante dans les cyanobactéries et notamment la spiruline (athrospira platensis) caractérisée en ce qu'il comprend une étape de culture des micro-algues dans un milieu de cultures alcalins contenant essentiellement un broyat de cellules néoplasiques ayant subies un choc thermique.

La culture de la micro-algue se faisant de façon monoclonale jusqu'à l'obtention d'une biomasse utile les cellules néoplasiques venant d'humains ayant subis tous les claviers viraux existants (HIV, hépatite B, hépatite C, herpès, mégalo-virus etc...).

L'invention a plus particulièrement pour objet un procédé d'induction physiologique et de rajout et d'intemalisation de molécules anti-cancéreuses caractérisées en ce qu'il comprend plusieurs étapes.

Notamment après la culture de micro-algues en présence de cellules néoplasiques remises en culture des dits cyanobactéries dans un milieu approprié de chlorure de sodium et de molécules anti-cancéreuses à une dilution de 1 à 5 millième. Ce procédé de rajout de molécules anti-cancéreuses spécifiques sera précédé de dilutions décroissantes dans le milieu de culture appropriée contenant une solution donc voici la composition en gr/litre :
o NaOHCO₃ 15,6
o K₂HPO₄ 0,6
o NaNO₃. 2,1
o K₂SO₄ 0,9
o NaCl. 1,6
o MgSO₄ 8
o HO₂. 0,2
o CaCl₂ 0,04
o FeSO₄ 7
o EDTA.. 0,08
o ZnSO₄ 7
o CuSO₄ 5
o NiSO₄ 0,047
o Qsp eau physiologique

Les molécules actives seront diluées dans cette solution, par exemple :
1/5000, 1/2500, 1/1250,1/625, 1/300, 1/100, 1/50, 1/10,1/5.

La cyanobactérie en contact décroissant avec les molécules anti-cancéreuses lèvera une éventuelle sélection de forme mutante facilement vérifiable puisqu'elle acceptera ces dites molécules ou un gène de résistance vérifiable par comparaison ou par simple cariotypage. La cyanobactérie aura alors une charge utile et utilisable pour le traitement des maladies (médecine humaine ou vétérinaire). Les doses pourront être ajustées en fonction du poids du patient et de la gravité de la maladie.

Le présente invention découle essentiellement du développement d'une éventuelle sélection de forme mutante spécifique et/ou d'un gène éventuel de résistance à un anti-mitotique donné ; car il ne peut y avoir d'intégration de molécules dits agressives sans avoir au préalable fait développer une éventuelle sélection de forme mutante.

La protéine de la micro-algue ayant reconnue plusieurs fois la protéine néoplasique sera reconnaître spécifiquement les cellules cancéreuses qu'elle aura pour cible. Cela aura un effet anti-corps récepteur. Ces protéines chargées d'anti-mitotiques feront imploser (en agissant sur les phases des différentes division cellulaire) les cellules cancéreuses sans atteindre (ou très peu) les autres cellules tout en évitant les pics plasmatiques et les effets secondaires dus à la chimiothérapie.

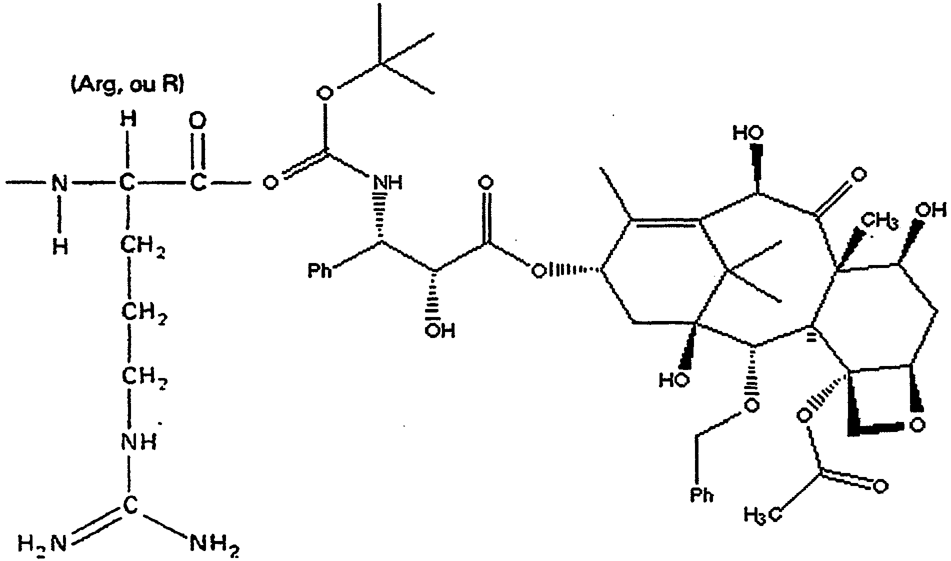

Exemple d'intégration d'une molécule active cyto-toxique comme le taxoter dans un acide aminé.

Pour faciliter cette intégration de la molécule anti-mitotique avec l'acide aminé, on soumettra alors le milieu de culture à l'action d'un champ électromagnétique pulsé de haute fréquence (1 GHz) de quelques kV.cm⁻¹ . Il s'en suivra que certaines portions de la molécule protéique vont entrer en résonance et tourner librement (relaxation dite δ). Les niveaux d'énergie d'activation des liaisons chimiques étant alors compatibles les liaisons des macromolécules s'en trouvent fortement facilitées.

## Revendications

1. Procédé de préparation de culture des cyanobactéries, et en particulier d'Arthrospira platensis, chargées en molécules anti-cancéreuses et présentant une résistance aux molécules anti-cancéreuses, caractérisé en ce ledit procédé comprend (i) une étape de culture de cyanobactéries dans un milieu de culture approprié contenant des sels minéraux et un broyat de cellules cancéreuses permettant une biomasse suffisante, puis (ii) une étape d'ajout dans le milieu de cultures des molécules anti-cancéreuses.

2. Procédé de préparation de culture des cyanobactéries et en particulier d'Arthrospira platensis selon la revendication 1, **caractérisé en ce que** le milieu de culture approprié est un milieu alcalin.

3. Procédé de préparation de culture des cyanobactéries et en particulier d'Arthrospira platensis selon la revendication 1 ou 2, **caractérisé en ce que** lesdites cellules cancéreuses ont subi un choc thermique.

4. Procédé de préparation de culture des cyanobactéries et en particulier d'Arthrospira platensis selon la revendication 1 à 3, **caractérisé en ce que** l'ajout des molécules anti-cancéreuses est réalisé par une dilution successive des molécules anti-cancéreuses dans ledit milieu de culture ; afin d'exprimer une éventuelle sélection de forme mutante de cyanobactéries qui représente une résistance à ces molécules anti-cancéreuses.

5. Procédé de préparation de culture des cyanobactéries et en particulier d'Arthrospira platensis selon l'une des revendications 1 à 4, **caractérisé en ce qu'**il comprend en outre (iii) une étape d'exposition du milieu de culture dans un champ électromagnétique pulsé de haute fréquence.

6. Application d'une culture des cyanobactéries et en particulier d'Arthrospira platensis obtenue par le procédé selon l'une des revendications 1 à 5 à la préparation de compositions pharmaceutiques à usage humain ou vétérinaire.

## Claims

1. Process for preparing a culture of cyanobacteria, in particular of Arthrospira platensis, loaded with anti-cancer molecules and presenting resistance to anti-cancer molecules, **characterized in that** said process comprises (i) a step of culturing cyanobacteria in a suitable culture medium containing mineral salts and a homogenate of cancer cells allowing a sufficient biomass, then (ii) a step of adding in the culture medium the anti-cancer molecules.

2. Process for preparing a culture of cyanobacteria and in particular of Arthrospira platensis according to claim 1, **characterized in that** the suitable culture medium is an alkaline medium.

3. Process for preparing a culture of cyanobacteria and in particular of Arthrospira platensis according to claim 1 or 2, **characterized in that** said cancer cells are heat shocked.

4. Process for preparing a culture of cyanobacteria and in particular of Arthrospira platensis according to claim 1 to 3, **characterized in that** the addition of anti-cancer molecules is carried out by a serial dilution of the anti-cancer molecules in said culture medium; in order to express an optional selection of mutant form of cyanobacteria which represents a resistance to said anti-cancer molecules.

5. Process for preparing a culture of cyanobacteria and in particular of Arthrospira platensis according to any one of claims 1 to 4, **characterized in that** it further comprises (iii) a step of exposing the culture medium to a pulsed high frequency electromagnetic field.

6. Application of a culture of cyanobacteria and in particular of Arthrospira platensis obtained by the process according to any one of claims 1 to 5 to the preparation of pharmaceutical compositions for human or veterinary use.

## Patentansprüche

1. Verfahren zur Herstellung einer Kultur von Cyanobakterien, und insbesondere von Arthrospira platensis, welche mit Anti-Krebs-Molekülen beladen sind und welche eine Resistenz gegenüber Anti-Krebs-Molekülen aufweisen, **dadurch gekennzeichnet, dass** das Verfahren die Schritte umfasst (i) Kultivieren von Cyanobakterien in einem geeigneten Kulturmedium umfassend Mineralsalze und ein Krebszellenhomogenat, welches eine ausreichende Biomasse ermöglicht, dann (ii) Hinzufügen der Anti-Krebs-Moleküle in das Kulturmedium.

2. Verfahren zur Herstellung einer Kultur von Cyanobakterien, und insbesondere von Arthrospira platensis nach Anspruch 1, **dadurch gekennzeichnet, dass** das geeignete Kulturmedium ein alkalisches Medium ist.

3. Verfahren zur Herstellung einer Kultur von Cyanobakterien, und insbesondere von Arthrospira platensis nach einem der Ansprüche 1 oder 2, **dadurch gekennzeichnet, dass** die Krebszellen einem Hitzeschock ausgesetzt wurden.

4. Verfahren zur Herstellung von Kulturen von Cyanobakterien, und insbesondere von Arthrospira platensis nach einem der Ansprüche 1 bis 3, **dadurch gekennzeichnet, dass** der Zusatz der Anti-Krebs-Moleküle durch eine serielle Verdünnung der Anti-Krebs-Moleküle in dem Kulturmedium durchgeführt wird; um eine mögliche Auswahl von einer mutierten Form von Cyanobakterien herzustellen, welche eine Resistenz gegenüber diesen Anti-Krebs-Molekülen aufweisen.

5. Verfahren zur Herstellung einer Kultur von Cyanobakterien, und insbesondere von Arthrospira platensis nach einem der Ansprüche 1 bis 4, **dadurch gekennzeichnet, dass** es weiterhin umfasst (iii) den Schritt des Aussetzens des Kulturmediums einem gepulsten elektromagnetischen Hochfrequenzfeld.

6. Verwendung einer Kultur von Cyanobakterien und insbesondere von Arthrospira platensis, erhalten durch das Verfahren nach einem der Ansprüche 1 bis 5 zur Herstellung von pharmazeutischen Zusammensetzungen für humane oder veterinäre Verwendung.
